# EUROPEAN PATENT SPECIFICATION

(11) **EP 1 480 663 B1**
(45) Date of publication and mention of the grant of the patent: **02.11.2006**
(21) Application number: 02745850.4
(22) Date of filing: 04.07.2002
(51) Int. Cl.: A61K 36/899, A61K 36/73

(54) **ANTIOBESTIC AND/OR ANTIDIABETIC AGENT CONTAINING CYANIDIN 3-GLUCOSIDE AS ACTIVE INGREDIENT**
MEDIKAMENT, ENTHALTEND CYANIDIN-3-GLYKOSID ALS WIRKSTOFF GEGEN FETTSUCHT UND DIABETES
AGENT ANTI-OBESITE ET/OU ANTIDIABETIQUE RENFERMANT DU 3-CYANIDINE GLUCOSIDE COMME PRINCIPE ACTIF

(30) Priority: 28.02.2002 JP 2002054286
(43) Date of publication of application: 01.12.2004
(73) Proprietor: SAN-EI GEN F.F.I., INC., Toyonaka-shi, Osaka 561-8588 (JP)
(72) Inventor: TSUDA, Takanori, Nagoya-shi, Aichi 468-0063 (JP); HORIO, Fumihiko, Nagoya-shi, Aichi 467-0048 (JP); OSAWA, Toshihiko, Nagoya-shi, Aichi 461-0023 (JP); UCHIDA, Koji, San-Ei Gen F.F.I., Inc., Toyonaka-shi, Osaka 561-8588 (JP); AOKI, Hiromitsu, San-Ei Gen F.F.I., Inc., Toyonaka-shi, Osaka 561-8588 (JP); KODA, Takatoshi, San-Ei Gen F.F.I., Inc., Toyonaka-shi, Osaka 561-8588 (JP)
(74) Representative: Sama, Daniele
(86) International application number: PCT/JP2002/006826
(87) International publication number: WO 2003/072121

(56) References cited:
- EP-A- 0 815 857
- WO-A-01/49281
- US-A- 4 229 439
- US-A1- 2001 016 573
- PATENT ABSTRACTS OF JAPAN vol. 1999, no. 13, 30 November 1999 (1999-11-30) & JP 11 218572 A (FUJI ELECTRIC CO LTD), 10 August 1999 (1999-08-10)
- JOHANSEN O-P ET AL: "CYANIDIN 3-Ä6-(P-COUMAROYL)-2-(XYLOSYL)-GLUCOSIDEÜ- 5-GLUCOSIDE AND OTHER ANTHOCYANINS FROM FRUITS OF SAMBUCUS CANADENSIS" PHYTOCHEMISTRY, PERGAMON PRESS, GB, vol. 30, no. 12, 1991, pages 4137-4141, XP001120128 ISSN: 0031-9422

## Description

### Technical Field

The present invention relates to the use of an antiobestic and/or antidiabetic agent containing cyanidin 3-glucoside as an active ingredient and a health food to which cyanidin 3-glucoside or a water-soluble extract from a plant containing cyanidin 3-glucoside is added.

### Background Art

Recently, as western diet becomes more prevailing, obesity keeps on increasing. The obesity is condition of adipose tissue increasing beyond its normal proportion in a body because of excessive intake of sugars (carbohydrates) and fats.

Normally, the sugars and fats, after being decomposed by digestive enzymes, are used as an energy source or the like. However, if taken excessively, an excess of sugars is changed into fatty acids via acetyl CoA and stored in tissue together with fats, resulting in obesity.

Adipose cells storing fats secret free fatty acids and a tumor necrosis factor (TNF) and inhibit the action of insulin which regulates metabolism of sugar including blood glucose, fats and amino acids. Therefore, if adipose cells increase owing to obesity, insulin does not act well and blood glucose increases to cause diabetes. It has been pointed out that the illness, if it hangs on for a long time, leads to peripheral vessel disorder and arterial sclerosis.

Anthocyanin pigments having a wide distribution in the plant kingdom are used as colorants, but are also known to have an decreasing effect on cholesterol in blood, for example, as bioacitivity (*Agric. Biol. Chem.,* **54**(1), 171-175, 1990).

Cholesterol is a constituent of biological membrane and serum lipoprotein and is synthesized *in vivo.* Normally, if cholesterol is externally taken, the amount of cholesterol synthesized *in vivo* is suppressed. However, if a large amount of cholesterol is taken, a cholesterol balance in the body is lost, which results in hypercholesterolemia. Excessive cholesterol causes arterial sclerosis. However, correlation between obesity and cholesterol is not known at present, and the control of cholesterol is not anticipated to lead to the control of weight.

Japanese Unexamined Patent Application Publication No. 2000-178295 described that cyanidin glucosides have an antitumor effect and that the intake of food containing cyanidin glucosides as active ingredients is preferable for prevention of or amelioration from diseases such as hyperlipidemia, arterial sclerosis, diabetes, fatty liver, myocardial infarction and the like. However, this reference presents pharmacological data supporting only the antitumor effect of the cyanidin glucosides, but does not present any pharmacological data about the antiobestic or antidiabetic effect thereof. It cannot be considered from medical findings that the antitumor effect results in amelioration from obesity or from the above-mentioned diseases.

### Disclosure of Invention

Under these circumstances, the present inventors have found that cyanidin 3-glucoside suppresses increase of adipose cells and reduces the blood glucose level, to accomplish the present invention.

Accordingly, the present invention provides the use of an antiobestic and/or antidiabetic agent containing cyanidin 3-glucoside as an active ingredient and a health food to which cyanidin 3-glucoside or a water-soluble extract from a plant containing cyanidin 3-glucoside is added.

### Brief Description of Drawings

Fig. 1 is a photograph of epididymal adipose tissue of group C (×200) in Example 2;
Fig. 2 is a photograph of epididymal adipose tissue of group A (×200) in Example 2;
Fig. 3 is a photograph of epididymal adipose tissue of group H (×200) in Example 2; and
Fig. 4 is a photograph of epididymal adipose tissue of group HA (×200) in Example 2.

### Best Mode for Carrying Out the Invention

In the present invention, cyanidin 3-glucoside can be obtained from a plant material, but may be obtained by a semisynthetic or whole synthetic method using a technique known in the field of art.

In the case where cyanidin 3-glucoside is to be obtained from a plant, may be used one material or two or more materials selected from the group consisting of purple corn, red cabbage, berries such as strawberry, boysenberry, raspberry, cranberry, blackberry and blueberry, grains such as wild rice, red rice, black rice and the like, tamarind, peanut, grape and hibiscus. Any portion of these plants, such as stems, leaves, roots, petals, fruits and seeds, or tissue culture cells thereof may be used. For example, kernels or cobs of purple corn, fruits of berries, pericarps and juice of grapes, and petals of hibiscus are preferably used.

The method of obtaining cyanidin 3-glucoside from a plant is not particularly limited. For example, since cyanidin compounds are stable under an acidic condition, the plant may be macerated in a warm or cool liquid under an acidic condition adjusted within the range of about pH 1 to pH 4 with use of an inorganic acid such as sulfuric acid or an organic acid such as citric acid, for 4 to 12 hours, for example, overnight to extract a pigment and form a water-soluble extract therefrom. The extract may be used as it is or may be filtered and used as filtrate.

For the extraction, the plant may be used directly or cut or crushed into an appropriate size. Alternatively, dried plants in these various shapes may be used.

The thus obtained water-soluble extract is thought to contain anthocyanin pigments such as cyanidin 3-glucoside as well as carbohydrates, salt and the like.

For this reason, the water-soluble extract may be refined to isolate cyanidin 3-glucoside by combining one or more treatments such as those using ion exchange resins or non-ionic adsorptive resins and filtration or by repeating a single treatment under the same or different conditions.

In the case where the extract is refined with a non-ionic adsorptive resin, may be used, for example, Duolite S-861, Duolite S-862, Duolite S-863 or Duolite S-866 of styrene series (trade names manufactured by Diamond Shamrock U.S.A.); Sepabeads SP70, Sepabeads SP700, Sepabeads SP825 or Sepabeads SP207 of aromatic series (trade names manufactured by Mitsubishi Chemical Corporation); Diaion HP10, Diaion HP20, Diaion HP21, Diaion HP40 or Diaion HP50 (trade names manufactured by Mitsubishi Chemical Corporation); Amberlite XAD-4, Amberlite XAD-7 or Amberlite XAD-2000 (trade names manufactured by Organo Corporation).

In the case where an ion exchange resin is used, may be used as a cation exchange resin, for example, Diaion SK 1B, Diaion SK 102, Diaion SK 116, Diaion PK 208, Diaion WK 10 or Diaion WK 20 (trade names manufactured by Mitsubishi Chemical Corporation), and may be used as an anion exchange resin, for example, Diaion SA 10A, Diaion SA 12A, Diaion SA 20A, Diaion PA 306, Diaion WA 10 or Diaion WA 20 (trade names manufactured by Mitsubishi Chemical Corporation).

The filtration may be ultrafiltration or reverse osmosis using various functional polymer membranes.

The inventors of the present invention have realized that cyanidin 3-glucoside obtained by the above-mentioned method suppresses increase of adipose cells and/or reduces the blood glucose level.

Accordingly, the present invention provides the use of an antiobestic and/or antidiabetic agent containing cyanidin 3-glucoside or a water-soluble plant extract containing cyanidin 3-glucoside as an active ingredient.

The agent is manufactured by a conventional method using a solid or liquid excipient known in the art. The solid excipients include, for example, lactose, sucrose, glucose, corn starch, gelatin, starch, dextrin, calcium phosphate, calcium carbonate, synthetic and natural aluminum silicate, magnesium oxide, dried aluminum hydroxide, magnesium stearate, sodium bicarbonate, dry yeast and the like. The liquid excipients include, for example, water, glycerin, propylene glycol, simple syrup, ethanol, ethylene glycol, polyethylene glycol, sorbitol and the like.

The above-described agent may contain conventional additives such as stabilizers including citric acid, phosphoric acid, malic acid and salts thereof; sweeteners of high potency including sucralose, acesulfame K and the like, and sweeteners such as sucrose, fructose and the like; preservatives including alcohols, glycerin and the like; demulcentias, diluents, buffers, flavoring agents and coloring agents, if desired. The agent may be manufactured into forms for internal use such as dispersion, tablet, emulsion, capsule, granule, chewable tablet, solution, syrup and the like by a conventional method or other suitable method.

The health food as used in the present invention signifies a food positively intended for health care, health maintenance and health enhancement compared with common food. The food added with cyanidin 3-glucoside or the water soluble plant extract containing cyanidin 3-glucoside according to the present invention can be the health food expected to exhibit effects of preventing, treating or ameliorating obesity and/or diabetes.

For example, the health food used for the above-mentioned purposes can be obtained by mixing or spraying cyanidin 3-glycoside or the water soluble plant extract containing cyanidin 3-glucoside of the invention with or into an intermediate product under processing or a final product, for example, solid, liquid or semisolid products usable itself as food such as starch, wheat flour, sugar and syrup; for example, bread, noodles, sweets including candies and cookies, other solid products; flavor enhancers such as dressings and sauces ; liquid products such as beverage, nutritional drink and soup ; semisolid products such as jerry.

As for the dose of cyanidin 3-glucoside of the invention, it is preferably used to an adult of 60 kg in an amount of 10 to 100 mg a day. However, the dose may vary depending on various factors such as health conditions of a person who will take cyanidin 3-glucoside, an administration method and a combination with other agents.

Cyanidin 3-glucoside of the present invention can be used safely and effectively as a substance for preventing, treating or ameliorating obesity and/or diabetes and, in additional to that, it has the feature of being able to be manufactured economically, because cyanidin 3-glucoside is contained in a lot of species of natural plants and has been used as a food additive.

### Examples

Hereinafter, the present invention will be detailed with reference to examples.

### Example 1:

To a mixture solution of 100 L of water and 450 g sulfuric acid (pH 2.3), 10 kg of dried purple corn (cobs and kernels) were added and allowed to stand overnight at room temperature to extract a pigment. After extraction, solid-liquid separation was performed using a metal gauze of 60 mesh. To the resulting liquid, was added a 2 % filter aid (Radiolite #700 manufactured by Showa Kagaku Kogyo Co., Ltd.) and filtration was carried out to obtain about 100 L of a crude extract of purple corn.

The crude extract was subjected to adsorption using 10 L of adsorptive resin (styrene vinyl benzene copolymer; Diaion HP20 manufactured by Mitsubishi Chemical Corporation) under the conditions of SV = 1 and a temperature of 20°C, and the resin was washed with 30 L (SV = 1) of water. Further, 15 L of 30 v/v% of ethanol aqueous solution containing 0.2 wt/v% of citric acid were passed through the resin (SV = 1, temperature : 20 °C), to collect eluate. The obtained eluate was concentrated under reduced pressure below 40 °C, to obtain a purple corn extract.

The purple corn extract was then passed through 10 L of Sepabeads SP207, adsorptive resin, (manufactured by Mitsubishi Chemical Corporation) under the conditions of SV = 1 and a temperature of 20°C, and the resin was washed with 30 L (SV = 1) of water and then with 10 L of 15 v/v% of ethanol aqueous solution containing 0.2 wt/v% of citric acid (SV = 1). Further, 20 L of 25 v/v% of ethanol aqueous solution containing 0.2 wt/v% of citric acid were passed through the resin (SV = 1, temperature : 20 °C), to collect eluate.

The eluate was concentrated under reduced pressure below 40 °C and then was spray-dried to obtain 70 g of a powdered purple corn extract of dark red color.

This powder contained 18.5 % of cyanidin 3-glucoside.

### Example 2 :

The action of cyanidin 3-glucoside was evaluated by adding the extract obtained in Example 1 to a normal diet (control diet) and to a diet containing fats in a higher proportion (high fat diet).

Four-week old C57BL/6J mice were allocated into four groups (6 to 8 mice per group), which were given the control diet (group C), the control diet + the extract obtained in Example 1 (group A), the high fat diet (group H) and the high fat diet + the extract obtained in Example 1 (group HA), respectively. The diets were taken freely for 12 weeks.

The control diet, 1 kg, was composed of 204 g of casein, 35 g of mixed minerals, 10 g of mixed vitamins, 2 g of choline chloride, 50 g of corn oil, 40 g of powered cellulose and 659 g of sucrose. The high fat diet, 1 kg, was composed of 204 g of casein, 35 g of mixed minerals, 10 g of mixed vitamins, 2 g of choline chloride, 50 g of corn oil, 40 g of powered cellulose, 300 g of lard, and 359 g of sucrose. In groups A and HA, the extract of Example 1 was added so that the content of cyanidin 3-glucoside was 0.2 % in the control diet and the high fat diet, and sucrose given as part of the diets was deducted by the amount of cyanidin 3-glucoside added. (1) Measurement of Weight and intake amount of diet

The amount of diet taken by each mouse a day was weighed 2, 4, 6, 10 and 12 weeks after the start of dietting the animals (Table 1).

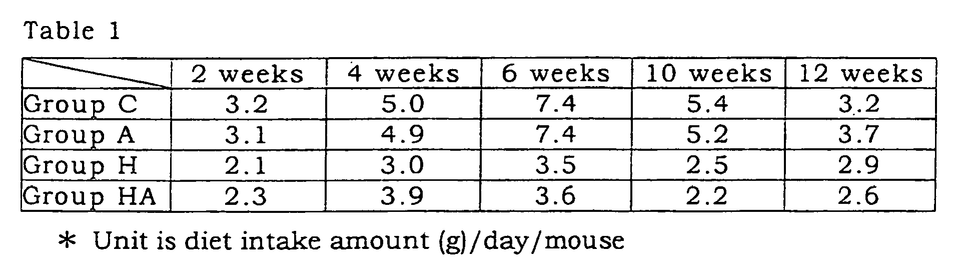

As shown in Table 1, since the high fat diet was high in calories, the intake amount of groups H and HA was smaller than that of groups C and A. However, no significant difference was observed in the intake amount between group C and group A or between group H and group HA.

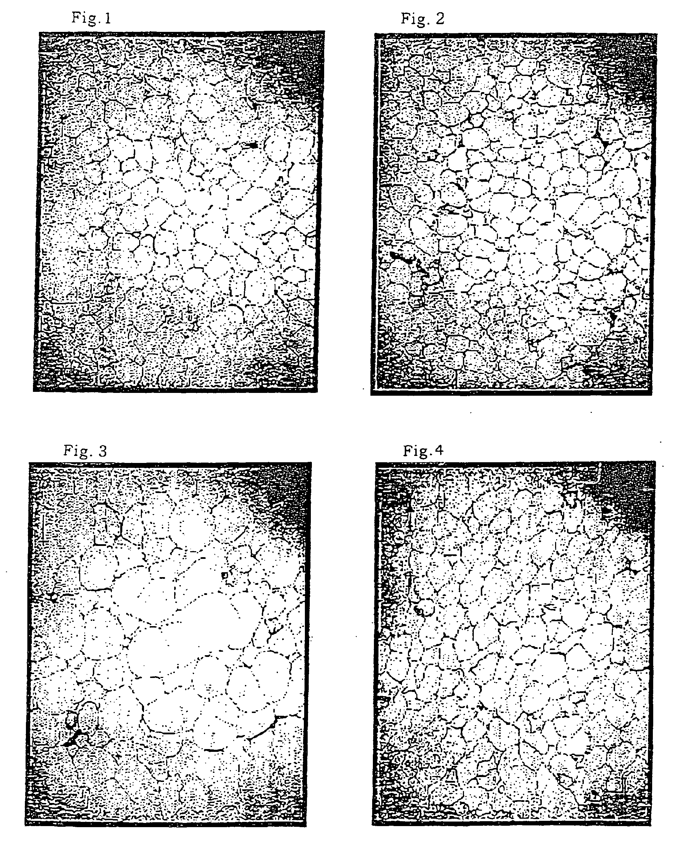

After 12 weeks, the mice were weighed and BMI (Body Mass Index = weight (kg) × body length (m)²) was calculated, and subcutaneous fat and epididymal peripheral fat were weighed (Table 2, values represent average ± standard error). Also epididymal adipose tissue was photographed (Figs. 1 to 4).

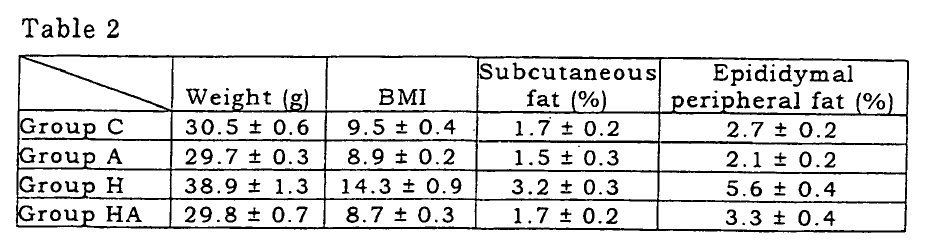

As shown in Table 2, group H showed significant increase in weight, BMI and adipose tissue amount as compared with group C, but group HA given the purple corn extract of Example 1 showed significant suppression in weight, BMI and adipose tissue amount. No significant difference was recognized among group C, group A and group HA.

As shown in Figs. 1 to 4, the size of epididymal adipose cells increased in group H. However, in group HA given the purple corn extract of Example 1, the size of adipose cells remained almost the same as that in group C, although group HA took the high fat diet. 2) Measurement of blood glucose level, serum insulin concentration and serum leptin concentration

Blood was collected from mice raised for 12 weeks. Glucose concentration, insulin concentration and leptin concentration in serum were measured with Glucose B - Test Kit (manufactured by Wako Junyaku Kogyo), Lbis® Insulin Kit (for mice - T, manufactured by Shibayagi Company) and Leptin Mouse Kit (manufactured by Morinaga Company), respectively (Table 3).

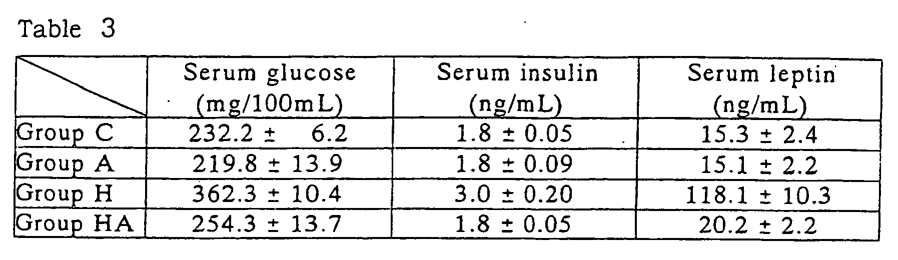

The concentration of serum glucose, serum insulin and serum leptin, which is a hormone having antiobestic action, increased significantly in group H as compared with group C. Group H was considered to have hyperinsulinemia and hyperleptinemia due to the intake of high fat diet.

Those values were remarkably decreased by dietting the purple corn extract of Example 1 (group HA). That is, the intake of the purple corn extract suppressed hyperglycemia and avoided hyperinsulinemia and hyperleptinemia. No significant difference was recognized in these concentrations among groups C, group A and group HA.

### (3) Measurement of mRNAs of fatty acid synthetases and of leptin

The increase of adipose cells involves increase of fatty acid synthetases and leptin at a molecular level. Therefore, the amount of expressed mRNAs of hepatic fatty acid synthetases and leptin was measured by RT-PCR.

Livers were taken out of mice raised for 12 weeks and immediately frozen in liquid nitrogen. Subsequently, the total RNAs were extracted from the livers using DNA and RNA extraction reagents and Isogen (manufactured by Nippon Gene) according to the conventional method and were subjected to the RT-PCR to obtain the results shown in Table 4.

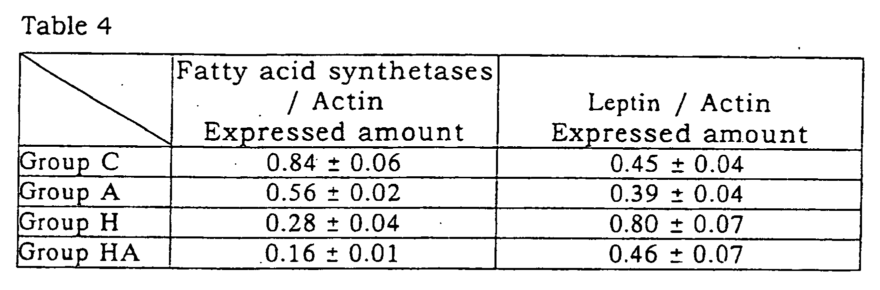

The amount of expressed mRNAs of the fatty acid synthetases was significantly smaller in group A than in group C, and was significantly smaller in group HA than in H. It was confirmed that the synthesis of fatty acids at the molecular level was affected by the purple corn extract of Example 1. The amount of expressed mRNAs of leptin was significantly larger in group H than in groups C, A and HA. Correlation between the amount of expressed mRNAs of leptin and the serum leptin concentration was confirmed.

Thus, it was shown that the purple corn extract containing cyanidin 3-glucoside did not suppress the increase of weight, fat and blood glucose level with regard to the ordinary diet but significantly suppresses it with regard to the high fat diet.

### Example 3 :

NOD mice which were an I type human diabetes model (purchased from Clea Japan Inc.) were administered with the extract obtained in Example 1 and the incidence of diabetes was examined.

Six-week-old female NOD mice were allocated into two groups, 16 mice per group, which were given a control diet (pellet CA-1 manufactured by Clea Japan Inc., group NC) and a control diet plus the extract obtained in Example 1 (group NA) for 24 weeks. The diets were taken freely. The incidence of diabetes was observed using dip stick sheets for urinary sugar (Table 5). In group NA, the extract of Example 1 was added so that the content of cyanidin 3-glucoside in the control diet was 0.2 %, and sucrose was deduced from the control diet by the amount of cyanidin 3-glucoside added.

**Table 5**

| Incidence of diabetes(%) | | | | | | | | | | | | | | | | | |
|---|---|---|---|---|---|---|---|---|---|---|---|---|---|---|---|---|---|
| Experiment diet intake weeks | 0-7W | 8W | 9W | 10W | 11W | 13W | 14W | 15W | 16W | 17W | 18W | 19W | 20W | 21W | 22W | 23W | 24W |
| Group NC | 0 | 6.2 | 12.5 | 12.5 | 12.5 | 12.5 | 18.7 | 18.7 | 18.7 | 18.7 | 31.1 | 31.1 | 43.5 | 43.5 | 50 | 50 | 50 |
| Group NA | 0 | 0 | 0 | 0 | 0 | 0 | 0 | 0 | 6.2 | 6.2 | 6.2 | 6.2 | 6.2 | 6.2 | 12.5 | 12.5 | 18.7 |

In group NA given the diet containing the purple corn extract of Example 1, the incidence of diabetes is significantly smaller as compared with group NC.

It was shown that the purple corn extract containing cyanidin 3-glucoside suppressed not only the incidence of diabetes when the high fat diet was given but also the incidence of type I diabetes.

### Example 4 :

Using the purple corn extract obtained in Example 1, materials were blended according to the following formula. The resulting mixture was filtrated, and was put in a 250-mL bottle and was sterilized at 60°C for 20 minutes to prepare a carbonated beverage containing 20 % of grape juice. The beverage, 250 mL, contained about 50 mg of cyanidin 3-glucoside.

| Unit (kg) | |
|---|---|
| Sucralose | 0.0136 |
| x5 concentrated transparent grape juice | 4.0 |
| Citric acid (crystalline) | 0.25 |
| Purple corn extract | 0.1 |
| Grape flavor | 0.10 |
| Fruit flavor | 0.10 |
| Water | proper amount |
| Total | 45.0 kg |

The above syrup was mixed with 55.0 kg carbonated water to make the total amount 100.0 kg.

### Example 5 :

Using the purple corn extract obtained in Example 1, materials were blended according to the following formula, and was prepared into tablets by a tablet machine. The tablets, 10 g, contained about 50 mg of cyanidin 3-glucoside.

| Unit (kg) | |
|---|---|
| Fine powdered glucose | 87.8 |
| 10 w/w% corn starch | 7.0 |
| Citric acid (crystalline) | 1.5 |
| Grape flavor | 1.0 |
| Purple corn extract | 2.7 |
| Total | 100.0 kg |

According to the present invention, the cyanidin compound naturally contained in a lot of species of plants can be utilized as antiobestic and/or antidiabetic agents for suppressing the increase of adipose cells and reducing blood glucose level safely and effectively.

## Claims

1. Use of cyanidin-3-glucoside as an active ingredient for preparation of an antiobestic and/or antidiabetic agent

2. Use according to claim 1, wherein cyanidin-3-glucoside is used as a water-soluble extract from a plant.

3. Use according to claim 2 wherein, the plant is purple corn.

4. Use according to claims 1-3, wherein the agent suppresses increase of adipose cells and/or reduces blood glucose level.

5. Use according to claims 1-4, wherein the agent is added to food.

## Patentansprüche

1. Verwendung eines Cyanidin-3-Glucosids als ein aktiver Bestandteil zur Bereitstellung eines Anti-Fettsucht- und/oder Anti-Diabetes-Wirkstoffes.

2. Verwendung nach Anspruch 1, worin Cyanidin-3-Glucosid als ein wasserlöslicher Extrakt aus einer Pflanze verwendet wird.

3. Verwendung nach Anspruch 2, worin die Pflanze lila Mais ist.

4. Verwendung nach Anspruch 1-3, worin der Wirkstoff einen Anstieg von Fettzellen unterdrückt und/oder einen Blutglucosespiegel senkt.

5. Verwendung nach Anspruch 1-4, worin der Wirkstoff zu Lebensmitteln hinzugefügt ist.

## Revendications

1. Utilisation de 3-cyanidine glucoside en tant que principe actif pour la préparation d'un agent anti-obésité et/ou antidiabétique.

2. Utilisation selon la revendication 1, dans laquelle le 3-cyanidine glucoside est utilisé en tant qu'extrait hydrosoluble d'une plante.

3. Utilisation selon la revendication 2, dans laquelle la plante est le maïs pourpre.

4. Utilisation selon les revendications 1-3, dans laquelle l'agent supprime l'augmentation des cellules adipeuses et/ou réduit le taux de glucose dans le sang.

5. Utilisation selon les revendications 1-4, dans laquelle l'agent est ajouté à des aliments.
